# EUROPEAN PATENT APPLICATION

(11) **EP 3 192 519 A2**
(43) Date of publication of application: **19.07.2017**
(21) Application number: 15809320.3
(22) Date of filing: 02.06.2015
(51) Int. Cl.: A61K 35/74, A61K 36/66, A61P 31/04, A61P 17/00

(54) **COMPOSITION CONTAINING PSEUDOMONAS AERUGINOSA CULTURE SOLUTION EXTRACT HAVING ANTIBIOTIC AND ANTISEPTIC ACTIVITIES, AND USE THEREOF**

(30) Priority: 20.06.2014 KR 20140075507
(71) Applicant: Joint Center For Biosciences, Incheon 406-840 (KR)
(72) Inventor: CHOI,Seung-Hyon, Yongin-si Gyeonggi-do 449-881 (KR); PARK, Ga In, Incheon, (21550) (KR)
(74) Representative: advotec.
(86) International application number: PCT/KR2015/005535
(87) International publication number: WO 2015/194772

(57) **Abstract**

The present invention provides an antiseptic and antibiotic composition containing a *Pseudomonas aeruginosa* culture solution extract as an active ingredient. The present invention provides a composition for preventing or treating acne, containing a *Pseudomonas aeruginosa* culture solution extract as an active ingredient. The composition containing a *Pseudomonas aeruginosa* culture solution extract, of the present invention, exhibits a broad antibiotic spectrum and a high antioxidant effect against various bacteria, and the composition can be applied to a cosmetic composition such as a cosmetic product and can also be useful for antibiotic, sterilizing and antiseptic purposes in various fields such as food, medical supplies, agricultural chemicals and household items. The present invention identifies a specific active ingredient causing the antibiotic effect of a *Pseudomonas aeruginosa* culture solution extract, and provides the structure thereof.

## Description

### Technical Field

The present application claims priorities from Korean Patent Application No. 10-2014-0075507 filed with the Korean Intellectual Property Office on 20 June 2014, the disclosures of which are incorporated herein by reference.

The present invention relates to a composition containing a *Pseudomonas aeruginosa* culture extract and having antibacterial and preservative activities, and a use thereof.

### Background Art

Paraben is a sterile preservative that is mainly used to suppress the growth of microorganisms and increase the preservation period in cosmetics, food, drugs, and the like. Paraben is known to have no toxicity, and thus has been used for a long time since it was first used for drugs in the mid 1920's. Paraben is widely used, and the use of paraben has been extended over a long period of time in various products. As studies have been reported on the estrogenicity of paraben, the problem of the safety of paraben on humans has been raised.

Studies on acute, sub-acute, and chronic toxicity of paraben have been conducted on the basis of various analysis results on experimental animals, and thus, sensitization reactions (such as skin inflammation by methyl paraben, and the retardancy, contact, and hypersensitiveness by methyl and propyl paraben) have been reported. As for the carcinogenicity of paraben, paraben has estrogenicity, and thus, the relationship between paraben and breast cancer through the use of cosmetics is presented. Anti-androgenicity of paraben is also known to interrupt the functions of male reproductive systems, and the endocrine disturbance effect of the paraben discharged as sewage on environments, including reproductive systems, has been discussed. The reason paraben is continuously used in various fields in spite of the potential harmfulness thereof is that the preservative power of natural ingredients is not as excellent as existing chemical synthetic preservatives. However, as the risk of the above-described paraben is reported, consumers express their repulsion of paraben preservative, and the request for substitute natural preservatives is increasing.

A biosurfactant, which is a surfactant obtained from biological materials, such as microorganisms, animals, and plants, receives attention since it has low toxicity, and is easily degraded by the ecosystem. The advantages of the biosurfactant over chemical synthetic surfactants are that, first, the biosurfactant is nontoxic and easily biodegraded, and thus the use of the biosurfactant is not a secondary source of contamination; second, the biosurfactant can be used for a particular purpose since it has a complex chemical structure, which is difficult to synthesize by an existing method; and third, the biosurfactant shows almost equal results to existing chemical synthetic surfactants in physical and chemical performances of the surfactant, such as surface tension degradation capacity, temperature, and the stability of pH.

In fact, the biosurfactant is used in various industrial fields in which chemical synthetic surfactants are mostly used, such as medicines, food, chemicals, detergents, secondary recovery of crude oil, pulp, paper making industry, the purification of oil contamination in the land and sea, degradation of milk fact in a treatment bath, and the like. In addition, research showed that the rhamnolipid-biosurfactant produced from *Pseudomonas sp.* changes the cell surface pattern to exhibit a pathogen inhibitory effect, and thus may be grafted as a new field of biomedicine. Therefore, research on the biosurfactant having the above advantages is actively being conducted globally, and many kinds of biosurfactants have been reported.

Prior to the present invention, a mixture type of biosurfactant containing rhamnolipid is extracted from the *Pseudomonas sp.* culture to measure the inhibitory effect against various pathogens, and as a result, it was verified that the biosurfactant had an excellent inhibitory effect against particular pathogens (acne-causing bacteria: *Propionibacterium acnes, Staphylococcus aureus*) compared with existing antibiotic agents. In addition, it was verified that paraben has a sterilizing effect against various pathogens at low concentrations, and on the basis of these results, paraben has been used as an existing cosmetic preservative. However, the development of eco-friendly and bio-friendly preservatives that can substitute for methyl paraben and the development of raw materials that inhibit acne-causing bacteria having antibiotic resistance to mitigate acne were studied, and the following test results may be specific contents and grounds for implementing the present invention.

Throughout the entire specification, many papers and patent documents are referenced and their citations are represented. The disclosure of the cited papers and patent documents are entirely incorporated by reference into the present specification, and the level of the technical field within which the present invention falls and the details of the present invention are explained more clearly.

### Detailed Description of the Invention

### Technical Problem

The present inventors endeavored to develop a preservative composition derived from a natural material, the composition being capable of effectively inhibiting the growth of microorganisms to increase the storage period of cosmetics or food. As a result, the present inventors established that a *Pseudomonas aeruginosa* culture extract exhibits an antibacterial effect against various pathogens, and further verified that the *Pseudomonas aeruginosa* culture extract of the present invention can be used as a cosmetic preservative and a food preservative by substituting for synthetic cosmetic substitutes of paraben, and thus the present inventors completed the present invention.

Therefore, an aspect of the present invention is to provide a preservative composition containing a *Pseudomonas aeruginosa* culture extract as an active ingredient.

Another aspect of the present invention is to provide an antibiotic composition containing a *Pseudomonas aeruginosa* culture extract as an active ingredient.

Another aspect of the present invention is to provide a composition for preventing or treating acne, containing a *Pseudomonas aeruginosa* culture extract as an active ingredient.

Still another aspect of the present invention is to provide an antioxidative composition containing a *Pseudomonas aeruginosa* culture extract as an active ingredient.

Another aspect of the present invention is to find out an active ingredient that causes an antibacterial effect of a *Pseudomonas aeruginosa* culture extract.

Other purposes and advantages of the present invention will be clarified by the following detailed description of the invention, claims, and drawings.

### Technical Solution

In accordance with an aspect of the present invention, there is provided a preservative composition containing a *Pseudomonas aeruginosa* culture extract as an active ingredient.

In accordance with another aspect of the present invention, there is provided an antibiotic composition containing a *Pseudomonas aeruginosa* culture extract as an active ingredient.

In accordance with another aspect of the present invention, there is provided a composition for preventing or treating acne, containing a *Pseudomonas aeruginosa* culture extract as an active ingredient.

In accordance with another aspect of the present invention, there is provided an antioxidative composition containing a *Pseudomonas aeruginosa* culture extract as an active ingredient

The present inventors endeavored to develop a preservative composition derived from a natural material, the composition being capable of effectively inhibiting the growth of microorganisms to increase the storage period of cosmetics or food. As a result, the present inventors established that a *Pseudomonas aeruginosa* culture extract exhibits an antibacterial effect against various pathogens, and further verified that the *Pseudomonas aeruginosa* culture extract of the present invention can be used as a cosmetic preservative and a food preservative by substituting for synthetic cosmetic substitutes of paraben.

The composition of the present invention contains a *Pseudomonas aeruginosa* culture extract as an active ingredient.

As used herein, the term "*Pseudomonas aeruginosa* culture extract" refers to one obtained by the extraction from a *Pseudomonas aeruginosa* culture.

According to the present invention, the *Pseudomonas aeruginosa* culture extract of the present invention contains rhamnolipid as a biosurfactant.

According to an embodiment of the present invention, the rhamnolipid contained in the composition of the present invention is di-rhamnolipid represented by chemical formula 1 or mono-Rhamnolipid represented by chemical formula 2:

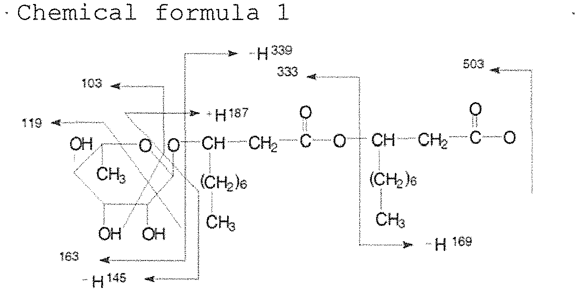

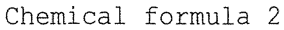

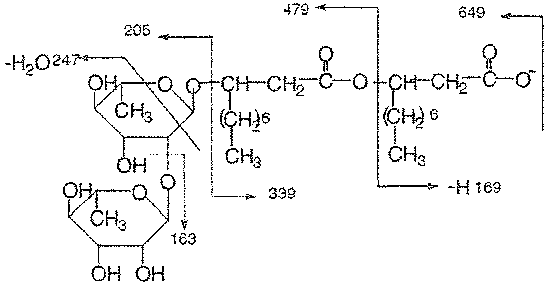

According to the present invention, the *Pseudomonas aeruginosa* culture extract has various structures of rhamnolipids, and of these, the Rhamnolipid of chemical formula 1 or 2 has significantly higher antibacterial activity compared with the other rhamnolipids extracted from the *Pseudomonas aeruginosa* culture extract.

According to an embodiment of the present invention, the *Pseudomonas aeruginosa* culture extract is obtained by inoculating and culturing *Pseudomonas aeruginosa* in a medium, centrifuging the culture to obtain a supernatant, adding a hydrochloric acid solution to the supernatant to perform precipitation, performing centrifugation to obtain only a precipitate, adding ethyl ether to the precipitate, and separating and concentrating only ethyl ether.

The extract of the present invention includes ones obtained by using the foregoing solvents, and also includes ones obtained by further applying a purification process thereto. For example, the extract of the present invention also includes fractions obtained by passing the extract through an ultrafiltration membrane with a cut-off value of a predetermined molecular weight, and fractions obtained through various purification methods that are further carried out, such as separation by various chromatographies (manufactured for separation depending on size, charge, hydrophobicity, or hydrophilicity).

The extract of the present invention may be prepared into a powder state by additional procedures, such as distillation under reduced pressure and freeze-drying or spray drying.

According to an embodiment of the present invention, in the composition of the present invention, the content of the *Pseudomonas aeruginosa* culture extract is 0.005-1.0 parts by weight on the basis of 100 parts by weight of the entire composition.

According to another embodiment of the present invention, in the composition of the present invention, the content of the *Pseudomonas aeruginosa* culture extract is 0.01-0.5 parts by weight on the basis of 100 parts by weight of the entire composition.

According to a particular embodiment of the present invention, in the composition of the present invention, the content of the *Pseudomonas aeruginosa* culture extract is 0.02-0.1 parts by weight on the basis of 100 parts by weight of the entire composition.

According to an embodiment of the present invention, the composition of the present invention has antibacterial activity and/or antifungal activity. The composition of the present invention exhibits widespread antibiotic activity against various microorganisms, especially, bacteria, fungi, and yeasts.

According to another embodiment of the present invention, the composition of the present invention exhibits antibiotic activity against at least one bacterium selected from the group consisting of *Staphylococcus spp., Bacillus spp., Pseudomonas spp., Candida spp., Propionibacterium spp., Streptococcus spp., Proteus spp., Corynebacterium spp., Enterococcus spp., Klebsiella spp.,* and *Escherichia coli.*

According to another embodiment of the present invention, the composition of the present invention has a high antioxidative effect, which has the same level as ascorbic acid (AA), which is a representative ingredient that has an antioxidative effect at a concentration of 0.5% or more.

As described above, the preservative/antibiotic composition of the present invention having widespread antibiotic spectra and antioxidative effects can be widely used to attain the purpose of antibacterial, sterilizing, disinfecting, and preservative effects in food, daily supplies, agricultural chemicals, medicines, and the like, as well as cosmetics.

According to the present invention, the preservative composition of the present invention may be prepared into a cosmetic composition. In cases where the composition of the present invention is prepared into a cosmetic composition, the Pseudomonas aeruginosa culture extract prevents the corruption of the cosmetic composition by bacteria, and thus can be used to substitute for a cosmetic preservative. In cases where the composition of the present invention is prepared into a cosmetic composition, the composition of the present invention may contain a *Pseudomonas aeruginosa* culture extract as the active ingredient, a salt thereof, or a solvate or hydrate thereof, and also ingredients that are ordinarily used in the cosmetic composition, and may contain, for example, ordinary adjuvants, such as an antioxidant, a stabilizer, a solubilizer, vitamins, a pigment, and a flavoring agent, and a carrier.

According to the present invention, the preservative composition of the present invention may be prepared into a food composition. In cases where the composition of the present invention is prepared into a food composition, the *Pseudomonas aeruginosa* culture extract prevents the corruption of the cosmetic composition by bacteria, and thus can be used to substitute for a food preservative.

In cases where the composition of the present invention is prepared into a food composition, the food composition contains the ingredients that are ordinarily added at the time of food manufacturing, as well as the *Pseudomonas aeruginosa* culture extract as an active ingredient, and may contain, for example, proteins, carbohydrates, fats, nutrients, seasonings, and flavoring agents. Examples of the foregoing carbohydrate may include ordinary sugars (monosaccharides, such as glucose and fructose; disaccharides, such as maltose, sucrose and oligosaccharides; and polysaccharides, such as dextrin and cyclodextrin) and sugar alcohols, such as xylitol, sorbitol, and erythritol. Examples of the flavoring agent may include natural flavoring agents (thaumatin, and stevia extract (e.g., rebaudioside A, glycyrrhizin, etc.)) and synthetic flavoring agents (saccharin, aspartame, etc.). For example, the food composition of the present invention, when prepared into a drink, may further contain citric acid, liquefied fructose, sugar, glucose, acetic acid, malic acid, fruit juice, a *Eucommia ulmoides* extract, a jujube extract, a licorice extract, and the like, in addition to the *Pseudomonas aeruginosa* culture extract of the present invention.

As used herein, the term "antibiotic composition" refers to a composition containing an antibiotic material that kills microorganisms including viruses, fungi, protozoa, and bacteria, or suppresses the growth thereof. Therefore, the antibiotic material has antibiotic activity, that is, antibacterial activity, antifungal activity, or antiviral activity.

The composition of the present invention exhibits a significant effect in the prevention and treatment of skin infection or acne caused by the bacteria. As validated in the following examples, the composition of the present invention significantly suppresses the growth of several bacteria, such as *Propionibacterium acnes,* which is well known as an acne-causing bacterium; *Staphylococcus aureus* and *Bacillus cereus,* which are gram positive bacteria; and *Pseudomonas aeruginosa* and *Candida albicans,* which are gram negative bacteria.

The composition for preventing or treating acne of the present invention may be prepared by containing a cosmetically effective amount of the *Pseudomonas aeruginosa* culture extract as an active ingredient of the present invention, and a cosmetically acceptable salt.

As used herein, the term "cosmetically effective amount" refers to an amount that is sufficient to attain an antibiotic effect against the microorganisms described in the present invention.

The composition for external application to skin of the present invention may be formulated into any dosage form that is ordinarily prepared, and examples thereof may include solution, suspension, emulsion, paste, gel, cream, lotion, powder, soap, surfactant-containing cleansing, oil, powder foundation, emulsion foundation, wax foundation, and spray, but are not limited thereto. More specifically, the cosmetic composition of the present invention may be formulated in a dosage form of emollient lotion, nourishing lotion, nourishing cream, massage cream, essence, eye cream, cleansing cream, cleansing foam, cleansing water, pack, spray, or powder.

In cases where the dosage form of the present invention is a paste, a cream, or a gel, an animal fiber, a vegetable fiber, wax, paraffin, starch, tragacanth, a cellulose derivative, polyethylene glycol, silicone, bentonite, silica, talc, or zinc oxide may be used as a carrier ingredient.

In cases where the dosage form of the present invention is a powder or a spray, lactose, talc, silica, aluminum hydroxide, calcium silicate, or a polyamide powder may be used as the carrier ingredient. Especially, in cases where the dosage form of the present invention is a spray, the spray may further include a propellant, such as chlorofluorohydrocarbon, propane/butane, or dimethyl ether.

In cases where the dosage form of the present invention is a solution or an emulsion, a solvent, a solubilizer, or an emulsifier may be used as the carrier ingredient, and examples of the carrier may include water, ethanol, isopropanol, ethyl carbonate, ethyl acetate, benzyl alcohol, benzyl benzoate, propylene glycol, 1,3-butyl glycol oil, glycerol fatty esters, polyethylene glycol, and fatty acid esters of sorbitan.

In cases where the dosage form of the present invention is a suspension, liquid diluents (such as water, ethanol, and propylene glycol), suspending agents (such as ethoxylated isostearyl alcohol, polyoxyethylene sorbitol ester, and polyoxyethylene sorbitan ester), microcrystalline cellulose, aluminum metahydroxide, bentonite, agar, or tragacanth may be used as a carrier ingredient.

In cases where the dosage form of the present invention is a surfactant-containing cleansing, aliphatic alcohol sulfate, aliphatic alcohol ether sulfate, sulfosuccinate monoester, isethionate, imidazolium derivatives, methyl taurate, sarcosinate, fatty acid amide ether sulfate, alkyl amido betaine, aliphatic alcohol, fatty acid glyceride, fatty acid diethanolamide, plant oil, lanoline derivatives, or ethoxylated glycerol fatty acid ester may be used as a carrier ingredient.

The ingredients contained in the composition for external application to skin of the present invention include ingredients that are usually used in the composition for external application to skin, in addition to the active ingredient and the carrier ingredient, and for example, the composition may contain ordinary adjuvants, such as an antioxidant, a stabilizer, a solubilizer, vitamins, a pigment, and a flavoring agent.

### Advantageous Effects

Features and advantages of the present invention are summarized as follows:
(i) The present invention provides a preservative and antibiotic composition containing a *Pseudomonas aeruginosa* culture extract as an active ingredient.
(ii) The present invention provides a composition for preventing or treating acne, containing a *Pseudomonas aeruginosa* culture extract as an active ingredient.
(iii) The composition containing the *Pseudomonas aeruginosa* culture extract of the present invention exhibits widespread antibiotic spectra and high antioxidative effects against various bacteria, and such a composition can be applied to a cosmetic composition, such as cosmetics, and furthermore, such a composition can be favorably used for the antibacterial, sterilizing, disinfecting, and preservative purposes in various fields, such as food, medicines, agricultural chemicals, and daily supplies.
(iv) It was established that the active ingredient of the the *Pseudomonas aeruginosa* culture extract of the present invention, causing a high antibacterial effect, includes rhamnolipids with particular structures (Rha-C10-C10, Rha-Rha-C10-C10) among various rhamnolipids, and thus, improved the efficiency of the industrial application of the rhamnolipids.

### Brief Description of the Drawings

Fig. 1 illustrates results confirming antibacterial effects of *Pseudomonas aeruginosa* culture extract (R1) through paper disk diffusion test.
FIG. 2 illustrates graphs showing CFU as a result of preservative power test (challenge test) on toner dosage form of products containing *Pseudomonas aeruginosa* culture extract.
Fig. 3 illustrates images after plate smearing in preservative power test on toner dosage form of products containing *Pseudomonas aeruginosa* culture extract.
Fig. 4 illustrates results obtained by measuring reduction rate of acne-related trouble according to concentration of *Pseudomonas aeruginosa* culture extract (Red bars represent a balm dosage form, and purple bars represent a cream dosage form).
Fig. 5 illustrates results obtained by confirming, through- photographing, the reduction of acne-related trouble after the composition containing *Pseudomonas aeruginosa* culture extract of the present invention is coated on skin of subjects.
Fig. 6 shows structures of rhamnose and rhamnolipids.
Fig. 7 illustrates analysis results of rhamnolipid through 1D 1H-NMR.
Fig. 8 illustrates proton analysis results of rhamnose.
Fig. 9 illustrates 2D-NMR analysis results.
Fig. 10 illustrates 2D NMR ROESY test results.
Fig. 11 illustrates results obtained by measuring relative density of rhamnose in sample through 1D 1H-NMR spectra preliminary analysis.
Fig. 12 illustrates antioxidative effect test results of *Pseudomonas aeruginosa* culture extract R1.
Fig. 13 illustrates test results of antibiotic activity of R1 and fractions isolated from R1 against S. *aureus.*
Fig. 14 illustrates LC analysis results of R1 and fractions isolated from R1.
Fig. 15 illustrates structure analysis results of active ingredient peak 1 and peak 2 utilizing LC-MS and LC-MS/MS.
Fig. 16 illustrates LC analysis results of R1.

### Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

### EXAMPLES

### Materials and Method

### 1. Production of biosurfactant

Prior to test, strain (CJM01) was selected. The results of identification showed *Pseudomonas aeruginosa.* The strain was inoculated in M9 medium (CaCl₂ 0.015 g/L, Na₂HPO₄ 6 g/L, KH₂PO₄ 3 g/L, NaCl 0.5 g/L, NH₄Cl 1 g/L, MgSO₄ 0.0625 g/L, glucose 20 g/L), and cultured at 37□ for 72 hours. After the culture, the culture was centrifuged to obtain cell-completely-removed culture. The cell-removed culture was lowered to pH 2 using HC1, and then precipitated at 4°C for one day. After the precipitation, centrifugation was performed to remove the supernatant and obtain only precipitate. The precipitate was extracted by ethyl ether, and then only ethyl ether layer was separated, followed by concentration using a rotary evaporator concentrator to remove all ethyl ether, thereby obtaining an extract (biosurfactants crude), which was named R1.

### 2. Antibacterial power test of extracted biosurfactant (paper disk diffusion test)

The mixture state of biosurfactant, extracted from microorganism (P. *aeruginosa)* culture, was named R1. Paper disk diffusion test was conducted on gram positive pathogens *(Staphylococcus aureus* and *Bacillus cereus)* and gram negative pathogens (*Pseudomonas aeruginosa* and *Escherichia coli*).

*S. aureus, B. cereus, P. aeruginosa,* and *E. coli* each were inoculated in Luria-Bertani (LB) medium (tryptone 10 g/L, NaCl 10 g/L, yeast extract 5 g/L), and liquid-cultured at 37°C in aerobic conditions for 18 hours. After the culture, 200 *µ*ℓ of each bacteria culture was plated on LB agar plate. R1 extracted from microorganism (*P. aeruginosa*) culture was dissolved at a concentration of 2 mg/ml in methanol. Methyl paraben, phenoxy ethanol, and Naturotics, as controls, were dissolved at a concentration of 10 mg/ml in methanol. 20 *µ*ℓ of the dissolved R1, methyl paraben, phenoxy ethanol, and Naturotics solutions each were put on paper disks (diameter: 6 mm), and then the paper disks were sufficiently dried. After that, the paper disks were placed on the agar plates plated with *S*. *aureus, B. cereus, P. aeruginosa,* and *E.coli* each. Each agar plate was cultured at 37°C in aerobic conditions for 18 hours. After 18 hours, the clear zones generated around the paper disks were observed.

### 3. Preservative power test of extracted biosurfactant (challenge test)

In order to investigate the possibility of R1 as a preservative compared with methyl paraben, the preservative power test was conducted by measuring the sterilizing power of a toner dosage form and a lotion dosage form of R1 against gram positive pathogens (*Staphylococcus aureus* and *Bacillus cereus*) and gram negative pathogens (*Pseudomonas aeruginosa* and *Escherichia coli*). The preservative power test was conducted on the basis of the U.S. Cosmetics, Toiletry, and Fragrance Association (CTFA), and the basis is that a particular material has a possibility as a preservative if the particular material in dosage forms of a toner or lotion kills 99.9% of inoculated bacteria within 7 days. Prior to the test, toner and lotion were manufactured, and the compositions thereof were shown in tables 1 and 2.

**[Table 1]**

| Toner composition | | | | | | | |
|---|---|---|---|---|---|---|---|
| | R1-0% | R1-0.01 %. | R1-0.05 % | R1 or Methyl paraben -0.1% | R1 or Methyl paraben -0.5% | R1 or Methyl paraben -1% | MeoH |
| Hyarluon ic acid | 2 g | 2 g | 2 g | 2 g | 2 g | 2 g | 2 g |
| Glycerin | 3 g | 3 g | 3 g | 3 g | 3 g | 3 g | 3 g |
| Aloewater | 40 g | 40 g | 40 g | 40 g | 40 g | 40 g | 40 g |
| Calendula extract | 2.8 g | 2.8 g | 2.8 g | 2.8 g | 2.8 g | 2.8 g | 2.8 g |
| Distilled water | 52. 2 g | 52.1 8 g | 52.1 g | 52 g | 51.2 g | 50.2 g | 50. 2 g |
| R1 or Methyl paraben (500 mg/ml MeOH) | 0 g | 20 µl | 100 µl | 200 µl | 1 ml | 2 ml | 0 |
| MeOH | 0 | 0 | 0 | 0 | 0 | 0 | 2 ml |
| Total | 100 g | 100 g | 100 g | 100 g | 100 g | 100 g | 100 g |

**[Table 2]**

| Lotion composition | | | |
|---|---|---|---|
| | R1 or Methyl paraben-0% | R1 or Methyl paraben-0.5% | MeOH |
| Aloewater | 82.5 g | 81.5 g | 81.5 g |
| Glycerin | 5 g | 5 g | 5 g |
| Sorbitan olivate | 1.5 g | 1.5 g | 1.5 g |
| Olive oil | 7 g | 7 g | 7 g |
| Hyarluronic acid | 2 g | 2 g | 2 g |
| Calendula extract | 2 g | 2 g | 2 g |
| R1 or Methyl paraben (500 mg/ml MeOH) | 0 | 1 ml | 0 |
| MeOH | 0 | 0 | 1 ml |
| Total | 100 g | 100 g | 100 g |

### 4. Test on toner dosage form against gram positive pathogens (Staphylococcus aureus and Bacillus cereus)

R1 dissolved in methanol was added to the prepared toner dosage form, so that the final concentration of R1 had 0%, 0.01%, 0.05%, and 0.1%, and the *S. aureus and B. cereus* cultures each were inoculated in each container. In addition, methyl paraben dissolved in methanol was added to the toner dosage form, so that the final concentration of the methyl paraben had 0%, 0.1%, 0.5%, and 1%, and the *S. aureus and B. cereus* cultures each were inoculated in each container. The number of inoculated bacterial cells was 10⁶-10⁷ colony forming unit (CFU)/ml. While the bacteria-inoculated toners were kept at room temperature, each toner was diluted immediately, one day, three days, five days, and seven days after the inoculation of bacteria, and 200 *µ*ℓ of the diluted toner was plated on LB agar plate. The death or not of bacteria was checked by calculating the number of cells and then comparing the calculated number with the number of initially inoculated bacteria. As control, only methanol was added to the toner dosage form, and the bacteria culture was inoculated, followed by repeated tests.

### 5. Test on toner dosage form against gram negative pathogen (Pseudomonas aeruginosa) and fungus (Candida albicans)

R1 and methyl paraben, which were dissolved in methanol, each were added to the toner dosage form, so that the final concentrations of R1 and the methyl paraben had 0%, 0.1%, 0.5%, and 1%, and the *P. aeruginosa, C. albicans* cultures each were inoculated in each container. The number of inoculated bacterial cells was 10⁶-10⁷ colony forming unit (CFU)/ml. While the bacteria-inoculated toners were kept at room temperature, each toner was diluted immediately, one day, three days, five days, and seven days after the inoculation of bacteria, -and 200 *µ*ℓ of the toner inoculated with P. *aeruginosa* was plated on LB agar plate, and 200 *µ*ℓ of the toner inoculated with C. *albicans* was plated on YPD agar plate. The death or not of bacteria was checked by calculating the number of cells and then comparing the calculated number with the number of initially inoculated bacteria. As control, only methanol was added to the toner dosage form, and the bacteria culture was inoculated, followed by repeated tests.
*YPD agar (peptone 5 g/L, yeast extract 5 g/L, dextrose 5 g/L, agar 15 g/L)

6. Test on lotion dosage form against gram positive pathogens (*Staphylococcus aureus* and *Bacillus cereus*), gram negative pathogen (*Pseudomonas aeruginosa*), and fungus (*Candida albicans*)

R1 and methyl paraben, which were dissolved in methanol, each were added to the toner dosage form, so that the final concentration of each of R1 and the methyl paraben had 0% and 0.5%, and the *S. aureus, B. cereus, P. aeruginosa,* and C. *albicans* cultures each were inoculated in each container. The number of inoculated bacterial cells was 10⁶-10⁷ colony forming unit (CFU)/ml. While the bacteria-inoculated lotions were kept at room temperature, each lotion was diluted immediately, one day, three days, five days, and seven days after the inoculation of bacteria, and 200 *µ*ℓ of the lotions inoculated with *S.* a*ureus, B. cereus,* and *P. aeruginosa* each were as plated on LB agar plate, and 200 *µ*ℓ of the lotion inoculated with C. *albicans* was plated on YPD agar plate. The death or not of bacteria was checked by calculating the number of cells and then comparing the calculated number with the number of initially inoculated bacteria. As control, only methanol was added to the lotion dosage form, and the bacteria culture was inoculated, followed by repeated tests.

### 7. Acne-related trouble reduction effect of products containing extracted biosurfactant

### 7-1. Kind, dose, and dosage form of products

Natural cosmetic products containing biosurfactant R1 extracted from microorganism (P. *aeruginosa)* culture were manufactured. The products were applied (5 g/ one person/ two weeks) to subjects having acne-related trouble symptoms (pustule acne, acne miliaris, acne scar, pimples, etc.), and then the acne-related trouble reduction effect was observed.

**[Table 3]**

| Kind of product | |
|---|---|
| - | Kind |
| 1 | Cream containing microorganism culture extract-0% |
| 2 | Cream containing microorganism culture extract-0.02% |
| 3 | Cream containing microorganism culture extract-0.1% |
| 4 | Balm containing microorganism culture extract-0% |
| 5 | Balm containing microorganism culture extract-0.02% |
| 6 | Balm containing microorganism culture extract-0.1% |
| 7 | Balm containing microorganism culture extract-0.5% |

**[Table 4]**

| Composition of balm dosage form product containing 0.5% R1 mixture | | |
|---|---|---|
| Ingrednet | g | % |
| Bees wax | 7 | 23.33333 |
| Tocopheryl acetate | 0.3 | 1 |
| Calendula extract | 0.1 | 0.333333 |
| Tea tree E.O | 0.3 | 1 |
| Sunflower seed oil | 0.15 | 0.5 |
| Rosemary leaf extract | | |
| Tocopherol | 0.15 | 0.5 |
| Grape E.O | 0.2 | 0.666667 |
| *Oenothera biennis* oil | 5 | 16.66667 |
| *Simmondsia chinensis* oil | 3 | 10 |
| Organic tamanu oil | 13.65 | 45.5 |
| R1 (Mixture) | 0.15 | 0.5 |
| Total | 30 | 100 |

**[Table 5]**

| Composition of balm dosage form product cotnaing 0.1% R1 mixture | | |
|---|---|---|
| Ingredient | g | % |
| Bees wax | 7 | 23.33333 |
| Tocopheryl acetate | 0.3 | 1 |
| Calendula extract | 0.1 | 0.333333 |
| Tea tree E.O | 0.3 | 1 |
| Sunflower seed oil | 0.15 | 0.5 |
| Rosemary leaf extract | | |
| Tocopherol | 0.15 | 0.5 |
| Grape E.O | 0.2 | 0.666667 |
| *Oenothera biennis* oil | 5 | 16.66667 |
| *Simmondsia chinensis* oil | 3 | 10 |
| Organic tamanu oil | 13.77 | 45.9 |
| R1 (Mixture) | 0.03 | 0.1 |
| Total | 30 | 100 |

**[Table 6]**

| Composition of balm dosage form product cotnaing 0.02% R1 mixture | | |
|---|---|---|
| Ingredient | g | % |
| Bees wax | 7 | 23.33333 |
| Tocopheryl acetate | 0.3 | 1 |
| Calendula extract | 0.1 | 0.333333 |
| Tea tree E.O | 0.3 | 1 |
| Sunflower seed oil | 0.15 | 0.5 |
| Rosemary leaf extract | | |
| Tocopherol | 0.15 | 0.5 |
| Grape E.O | 0.2 | 0.666667 |
| *Oenothera biennis* oil | 5 | 16.66667 |
| *Simmondsia chinensis* oil | 3 | 10 |
| Organic tamanu oil | 13.77 | 45.9 |
| R1 (Mixture) | 0.03 | 0.1 |
| Total | 30 | 100 |

**[Table 7]**

| Composition of balm dosage form product cotnaing 0% R1 mixture | | |
|---|---|---|
| Ingredient | g | % |
| Bees wax | 7 | 23.33333 |
| Tocopheryl acetate | 0.3 | 1 |
| Calendula extract | 0.1 | 0.333333 |
| Tea tree E.O | 0.3 | 1 |
| Sunflower seed oil | 0.15 | 0.5 |
| Rosemary leaf extract | | |
| Tocopherol | 0.15 | 0.5 |
| Grape E.O | 0.2 | 0.666667 |
| *Oenothera biennis* oil | 5 | 16.66667 |
| *Simmondsia chinensis* oil | 3 | 10 |
| Organic tamanu oil | 13.8 | 46 |
| R1 (Mixture) | 0 | 0 |
| Total | 30 | 100 |

**[Table 8]**

| Composition of cream dosage form product cotnaing 0% R1 mixture | | |
|---|---|---|
| Material | g | % |
| Witch hazel water | 18.5 | 37 |
| Golden *Simmondsia chinensis* oil | 3 | 6 |
| *Oenothera odorata* oil | 2.5 | 5 |
| Olive-emulsified wax | 2.5 | 5 |
| *Aloe vera* leaf extract | 18 | 36 |
| Glycerine | | |
| Glycerylacrylate/acrylic acid copolymer | | |
| Propylene glycol | | |
| *Centella asiatica* extract | 3 | 6 |
| Tocopheryl acteate | 1 | 2 |
| Tee tree essencial oil | 0.5 | 1 |
| Grafefruit essential oil | 0.5 | 1 |
| Natural medicinal herb preservative | 0.5 | 1 |
| R1 (mixture) | 0 | 0 |
| Total | 50 | 100 |

**[Table 9]**

| Composition of cream dosage form product cotnaing 0.02% R1 mixture | | |
|---|---|---|
| Material | g | % |
| Witch hazel water | 18.49 | 36.98 |
| Golden *Simmondsia chinensis* oil | 3 | 6 |
| *Oenothera odorata* oil | 2.5 | 5 |
| Olive-emulsified wax | 2.5 | 5 |
| *Aloe vera* leaf extract | 18 | 36 |
| Glycerine | | |
| Glycerylacrylate/acrylic acid copolymer | | |
| Propylene glycol | | |
| *Centella asiatica* extract | 3 | 6 |
| Tocopheryl acteate | 1 | 2 |
| Tee tree essencial oil | 0.5 | 1 |
| Grafefruit essential oil | 0.5 | 1 |
| Natural medicinal herb preservative | 0.5 | 1 |
| R1 (mixture) | 0.01 | 0.02 |
| Total | 50 | 100 |

**[Table 10]**

| Composition of cream dosage form product cotnaing 0.1% R1 mixture | | |
|---|---|---|
| Matrial | g | % |
| Witch hazel water | 18.45 | 36.9 |
| Golden *Simmondsia chinensis* oil | 3 | 6 |
| *Oenothera odorata* oil | 2.5 | 5 |
| Olive-emulsified wax | 2.5 | 5 |
| *Aloe vera* leaf extract | 18 | 36 |
| Glycerine | | |
| Glycerylacrylate/acrylic acid copolymer | | |
| Propylene glycol | | |
| *Centella asiatica* extract | 3 | 6 |
| Tocopheryl acteate | 1 | 2 |
| Tee tree essencial oil | 0.5 | 1 |
| Grafefruit essential oil | 0.5 | 1 |
| Natural medicinal herb preservative | 0.5 | 1 |
| R1 (mixture) | 0.05 | 0.1 |
| Total | 50 | 100 |

### 7-2. Subjects

The present study was directed to observational study clinical tests of control and test groups by a single center, and the purpose of the present study was to evaluate the effectiveness and safety of products, such as reducing acne-related trouble symptoms, when the products were applied to subjects having acne-related troubles.

Primary endpoint is the reduction rate of acne-related trouble symptoms two weeks after the use of the products, and it was evaluated whether there is a difference in the reduction rate of acne-related trouble symptoms between before and after testing. The total number of subjects was set to 30 in consideration of a drop-out rate of about 10%, on the basis of clinical tests carried out in the past. Three subjects who did not sign a consent form for personal reasons did not participate in the present clinical test, and the total number of subjects participating in the present clinical test was 27. The drop-out rate was 0%, and thus, the total number of subjects who received final evaluations was 27.

### 7-3. Methods

5 g of test product was used for two weeks, and a cream or balm type product was applied to the skin lesion site designated by a tester twice or more (morning, evening, frequently) every day for two weeks.

Participants of the clinical test for evaluating acne-related trouble cosmetic evaluations were collected from students of Inha University and Incheon University, and the clinical test was conducted on voluntary applicants who were agreeable to the standard of application and were not agreeable to the standard of exception. All subjects were selected through screening. A total of 27 subjects agreed to participate in the test, and 16 were men and 11 were women. The ages of the subjects were distributed between 20 and 28. The number of drop-out subjects was zero, and the number of final subjects who completed evaluations was 27.

For the evaluation of acne-related trouble reduction effect, at every visit, visual evaluation, and image photographing were conducted, and a survey containing subjective opinions of the subject, including directly checking the extent of change (extent of feeling change and absence or presence of abnormal responses) every day, was conducted. For the visual evaluation, at day 0 of the clinical test, the number of ongoing acnes on the lesion site, which was designated by the tester before the product was applied, was set as an initial point, and the reduction degree by naked eyes at such a lesion site two weeks after testing was digitalized. The lesion site of each subject participating in the clinical test was image-photographed before testing and at every visit for clinical test measurement. In addition, the survey about subjective opinions of the subject was conducted

### 7-4. Effect evaluation standards, evaluation methods, and analysis methods

Subjects suitable for the standard of selection and subjects who used supplied products for 2 weeks were selected as effectiveness evaluation subjects. On weeks 0, 1, and 2, a visual evaluation with respect to acne-related trouble reduction, such as checking the number of ongoing acnes and photographing images, was conducted, and a survey containing subjective opinions of the subjects was conducted. Summary and analysis of test results were conducted by statistical estimation scheme.

### 8. NMR analysis

NMR analysis was performed on biosurfactant R1 extracted from microorganism (P. *aeruginosa)* culture, biosurfactants R2 and R3 obtained by changing culturing and extraction manners with respect to the same P. *aeruginosa,* and biosurfactant R90 containing 90% of rhamnolipid (one kind of biosurfactant), purchased from SIGMA-ALDRICH.

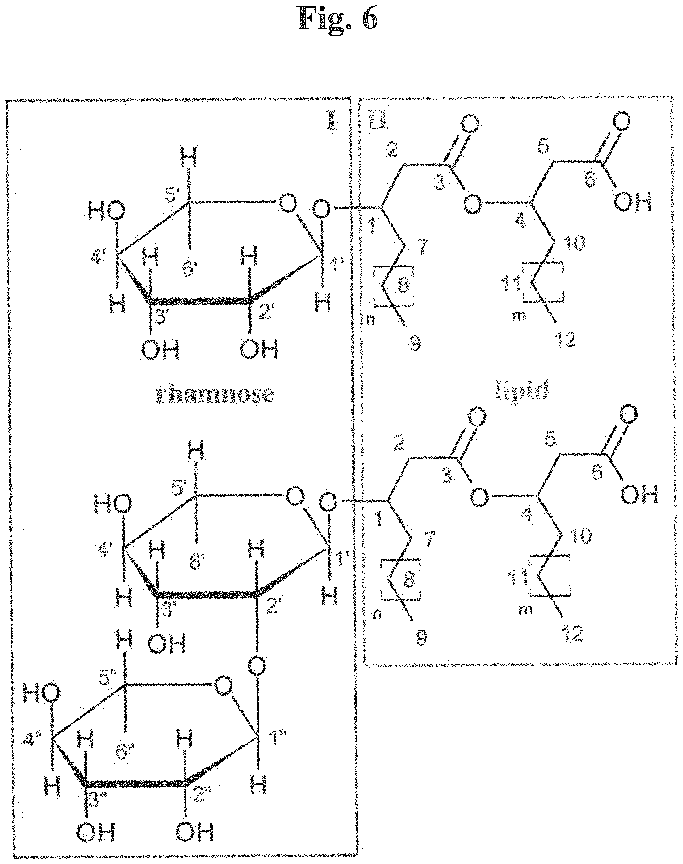

The structure of rhamnolipid contains one or two rhamnose (sugar) moieties (left panel of Fig. 6) and lipid moiety containing two aliphatic chains (right panel of Fig. 6). The diversity of the rhamnolipid structure is due to the number of rhamnose rings, one or two, and the structures (length and saturation degree) of two aliphatic chains. Four different biosurfactant extracts (samples 0, 1, 2, and 3) and commercially accessible rhamnolipid (sample 90) each were dissolved in chloroform-methanol mixture (2:1), followed by NMR analysis.

### 9. Antioxidative effect of Pseudomonas aeruginosa culture extract

For the application of *Pseudomonas aeruginosa* culture extract as a preservative, the test on an antioxidative effect thereof was conducted.

DPPH radical shows purple, and reacts with an antioxidant to show yellow. The color change of the DPPH radical is distinctive as it reacts with a reagent having excellent antioxidative ability. R1, ascorbic acid (AA), and methyl paraben (MTP) each were dissolved at different concentrations in methanol. The thus obtained samples (100 *µ*ℓ) each were dissolved at a concentration of 0.006% in methanol, and mixed with 100 *µ*ℓ of the DPPH radical solution to prepare a total volume of 200 *µ*ℓ, followed by reaction in a light-blocked space for 30 minutes. After completion of the reaction, the absorbance of each mixture was measured at 517 nm to determine antioxidative ability of each sample.

### 10. Search of active ingredients causing antibiotic effect of Pseudomonas aeruginosa culture extract

The *Pseudomonas aeruginosa* culture extract is in a mixture state of several ingredients rather than a single ingredient. Tests for searching an active ingredient causing an antibiotic effect, among various ingredients contained in the *Pseudomonas aeruginosa* culture extract, and discovering the structure thereof were conducted.

### Separation of mixture state of R1 extracted from microorganism (P. aeruginosa) culture

1 mg of a mixture state of R1 extracted from a microorganism (*P. aeruginosa*) culture was dissolved in methanol, and then injected in the sep-Pak vac 1cc tc18 cartridge column. After that, the column was washed with solvent A, and solvent B as a development solvent was injected thereinto. Solvent B with different concentrations was injected into the column, and the fractions passing through the column were collected, and named fraction 1, fraction 2, fraction 3, fraction 4, fraction 5, and fraction 6. The different concentrations of solvent B injected to the column were 50%, 55%, 60%, 65%, 70%, and 75%. The compositions of solvents A and B are shown in table 11.

**[Table 11]**

| Compositions of solvents A and B | |
|---|---|
| Solvent A | 0.1% Formic acid in water |
| Solvent B | 0.1% Formic acid in ACN |

### Analysis of antibiotic activity of fractions separated from R1

Each of the obtained fractions was dried, and then dissolved at a concentration of 10 mg/ml in methanol. 20 *µ*ℓ of each of the fractions dissolved in methanol was dropped on an agar plate plated with the S. *aureus* culture, and cultured at 37□ for 18 hours. After 18 hours, the clear zone generated on the agar plate was observed.

### Analysis of structures of fractions separated from R1 through LC/MS

LC-MS for structural analysis of the separated fractions was conducted using 1.8 µm 2.1 * 100 mm SB-Aq RRHD (analysis column) and 2.1 * 5 mm 1.8 µm Zorbax SB-C8 (guard column) in a negative mode of ion polarity. The separated fractions were dissolved in methanol to prepare samples. 20 *µ*ℓ of each sample was injected into HPLC equipped with the columns, and then was analyzed, together with a mass spectrometer connected thereto. In HPLC, a mixture of solvent A and solvent B was used as a development solvent, and the initial mixing percent of solvent A and solvent B was 50%. The sequential order was as follows. 100% solvent B (19 min), 100% solvent B (20.5 min), 50% solvent B (21 min), 50% solvent B (25 min), HPLC flow rate: 0.15 ml/min

The scanned mass range was 75~1000 m/z. MS/MS was conducted on a particular ingredient causing an antibiotic effect.

### Results

### 1. Test on antibiotic power of extracted biosurfactant

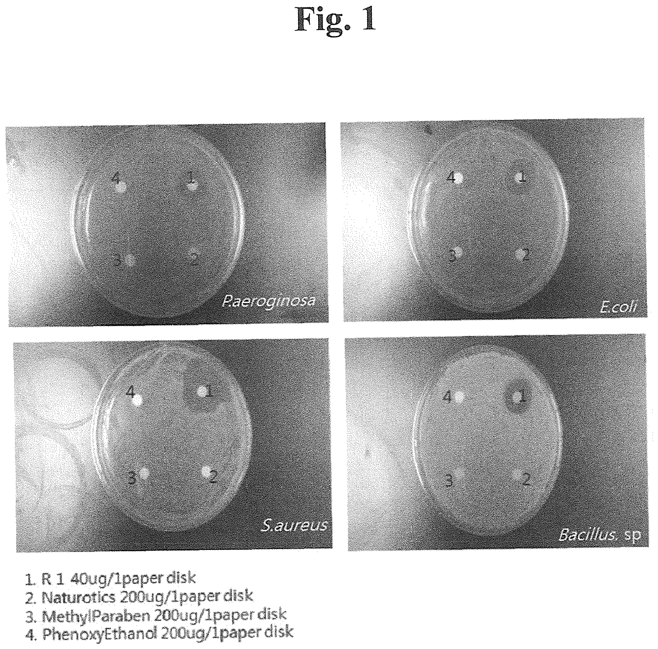

The mixture state of biosurfactant, extracted from microorganism (*P. aeruginosa*) culture, was named R1. The antibiotic power test was conducted on gram positive pathogens (*Staphylococcus aureus* and *Bacillus cereus*) and gram negative pathogens (*Pseudomonas aeruginosa* and *Escherichia coli*). As a result of the paper-disk diffusion test, R1 was a significantly large clear zone at a concentration, which was five-fold smaller than those of methyl paraben, phenoxy ethanol, and Naturotics (Fig. 1).

### 2. Test on preservative power of extracted biosurfactant

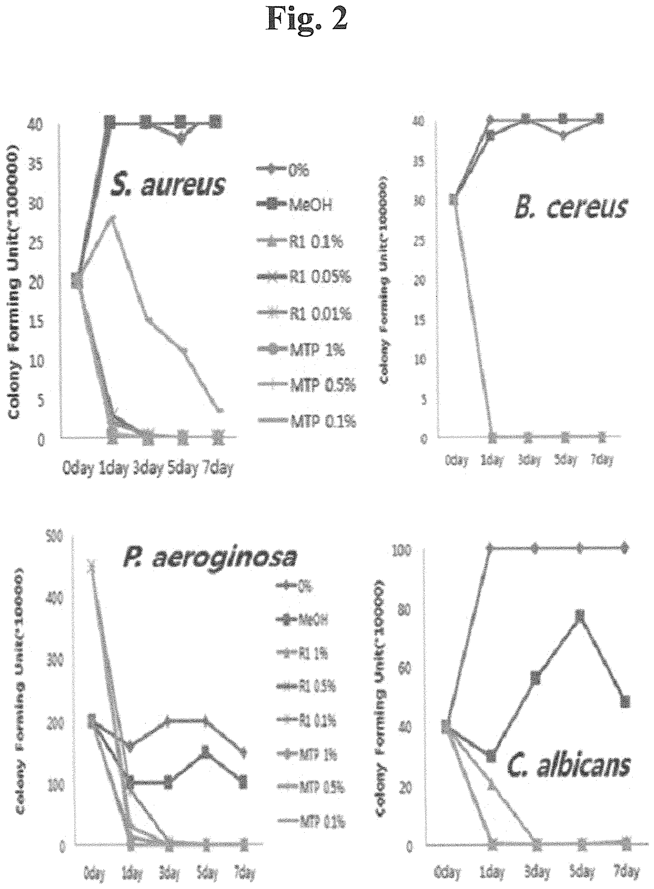

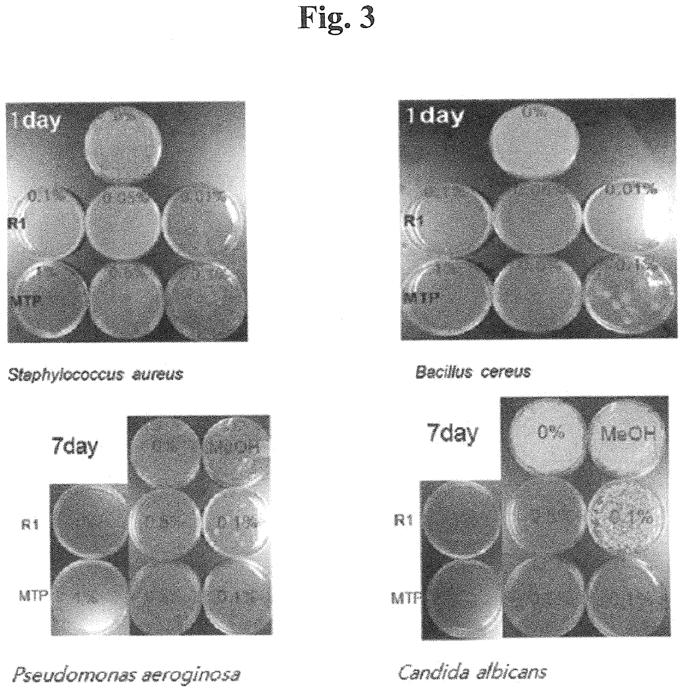

As a result of the preservative power test, in a toner dosage form, R1 killed 99.9% of the inoculated bacteria within 7 days against all the inoculated bacteria, and especially, R1 killed 99.9% of the inoculated bacteria at a concentration of 0.01%, which was 10-fold smaller than that of methyl paraben, in only one day against gram positive pathogens *Staphylococcus aureus* and *Bacillus cereus.* R1 killed 99.9% of the inoculated bacteria at a concentration of 0.5%, which was the same concentration as methyl paraben, within five days against gram negative pathogen (P*seudomonas aeruginosa*) and fungus (C*andida albicans*) (Figs. 2 and 3). In addition, in a lotion dosage form, R1 killed 99.9% of the inoculated bacteria at a concentration of 0.5%, which was the same concentration as methyl paraben, within seven days against all the inoculated bacteria (table 12). These results show that R1 has a possibility as a preservative.

**[Table 12]**

| Preservative power test of R1 in dosage form (%-survival rate of inoculated bacteria) | | | | | | |
|---|---|---|---|---|---|---|
| | | Day 0 | Day 1 | Day 3 | Day 5 | Day 7 |
| *Staphylococcusaureus* | 0% | 100% | 6.74% | 0.06% | 30% | 78% |
| | MeOH | 100% | 15.70% | 4.27% | 0.04% | 0.04% |
| | R10.5% | 100% | 0% | 0% | 0% | 0% |
| | MTP0.5% | 100% | 0% | 0% | 0% | 0% |
| *Bacillus cereus* | 0% | 100% | 0.09% | 0.23% | 35% | 54% |
| | MeOH | 100% | 0.17% | 0.02% | 0.02% | 0.02% |
| | R10.5% | 100% | 0.01% | 0% | 0% | 0% |
| | MTP0.5% | 100% | 0.12% | 0.06% | 0.02% | 0.01% |
| *Pseudomonasaeruginosa* | 0% | 100% | 0.30% | 0.60% | 0.02% | 0.02% |
| | MeOH | 100% | 7.30% | 0.80% | 0.03% | 0.02% |
| | R10.5% | 100% | 7.30% | 0.60% | 0.006% | 0% |
| | MTP0.5% | 100% | 0% | 0% | 0% | 0% |
| *Candida albicans* | 0% | 100% | 37.50% | 1% | 0.5% | 0.5% |
| | MeOH | 100% | 27.50% | 1.80% | 0.4% | 0.3% |
| | R10.5% | 100% | 16.30% | 0.10% | 0% | 0% |
| | MTP0.5% | 100% | 0% | 0% | 0% | 0% |

### 3. Acne-related trouble reduction effect of products containing extracted biosurfactant

Natural cosmetic products containing biosurfactant R1 extracted from the microorganism (*P. aeruginosa*) culture were manufactured, and then applied to subjects having acne-related trouble symptoms. As a result, the acne-related trouble reduction rate was significantly increased in the subjects using products containing R1 rather than the subjects not containing R1, and abnormal skin responses (itching, flushing, red marks, swelling, furuncles, prick, etc.) were not observed in all the subjects.

**[Table 13]**

| Acne-related trouble reduction rate | | | |
|---|---|---|---|
| Subject No. | Gender | Extract content and product dosage form | Reduction rate |
| 1 | Female | 0.5% / Balm | 57% |
| 2 | Male | 0.1% / Balm | 57% |
| 3 | Male | 0.02% / Cream | 75% |
| 4 | Male | 0.02% / Balm | 75% |
| 5 | Male | 0% / Cream | 60% |
| 6 | Female | 0% / Balm | 47% |
| 7 | Female | 0.1% / Balm | 43% |
| 8 | Female | 0.5% / Balm | 50% |
| 9 | Female | 0% / Cream | 20% |
| 10 | Female | 0.1% / Balm | 100% |
| 11 | Female | 0.02% / Balm | 50% |
| 12 | Female | 0% / Cream | 38% |
| 13 | Male | 0.1% / Cream | 54% |
| 14 | Male | 0.02% / Balm | 100% |
| 15 | Male | 0.1% / Cream | 80% |
| 16 | Female | 0.02% / Cream | 88% |
| 17 | Female | 0% / Cream | 31% |
| 18 | Female | 0.02% / Balm | 50% |
| 19 | Male | 0.1% / Balm | 64% |
| 20 | Male | 0.1% / Balm | 60% |
| 21 | Male | 0.02% / Cream | 63% |
| 22 | Male | 0.02% / Balm | 57% |
| 23 | Male | 0.1% / Balm | 60% |
| 24 | Male | 0.5% / Balm | 60% |
| 25 | Male | 0.5% / Balm | 65% |
| 26 | Male | 0.5% / Balm | 100% |
| 27 | Male | 0.02% / Cream | 100% |

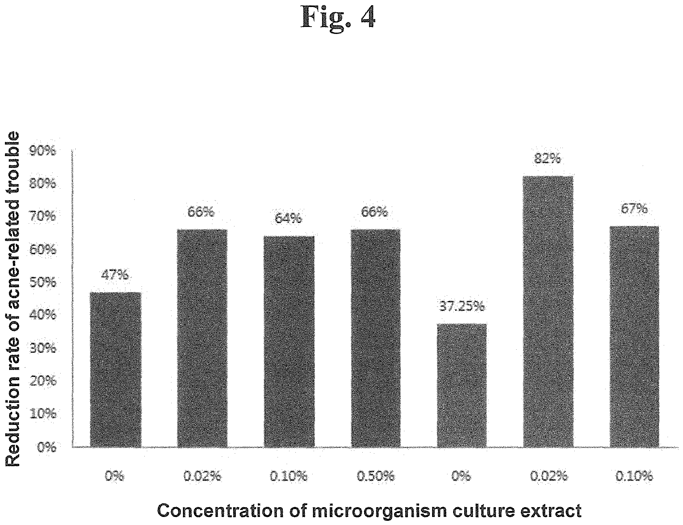

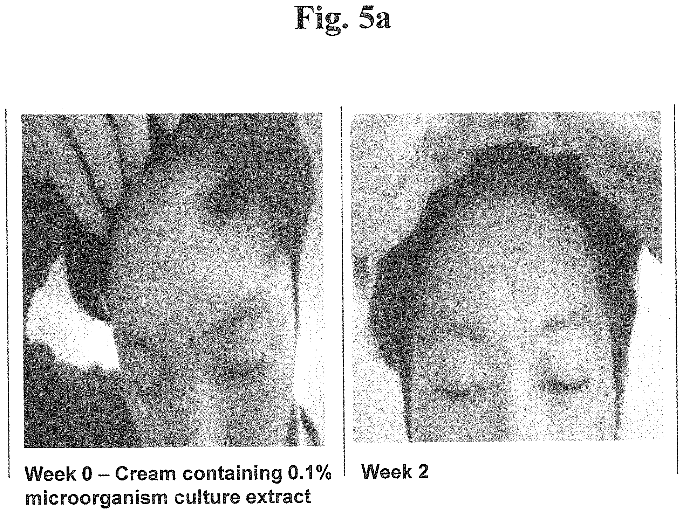

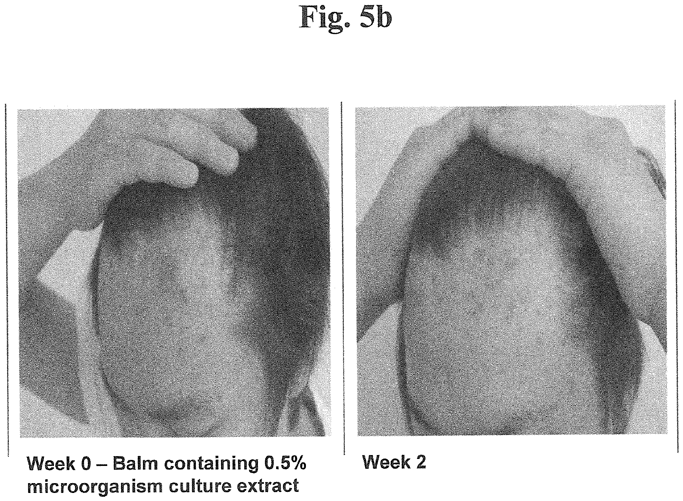

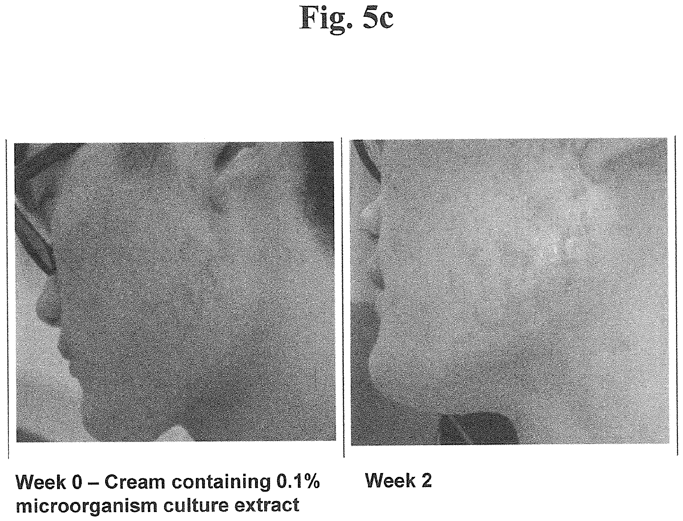

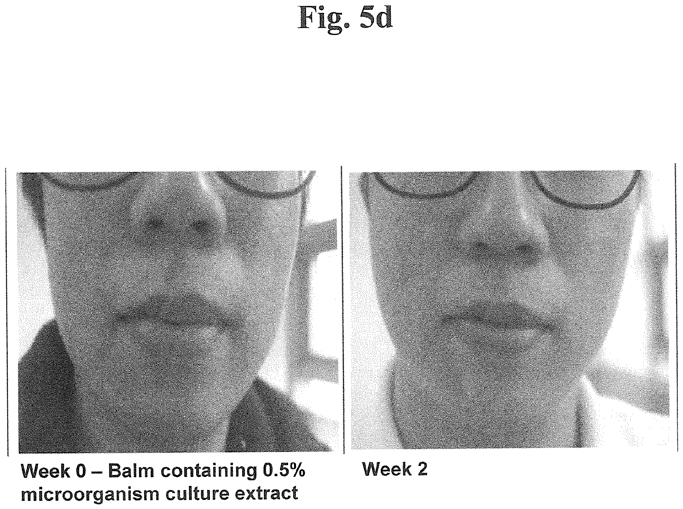

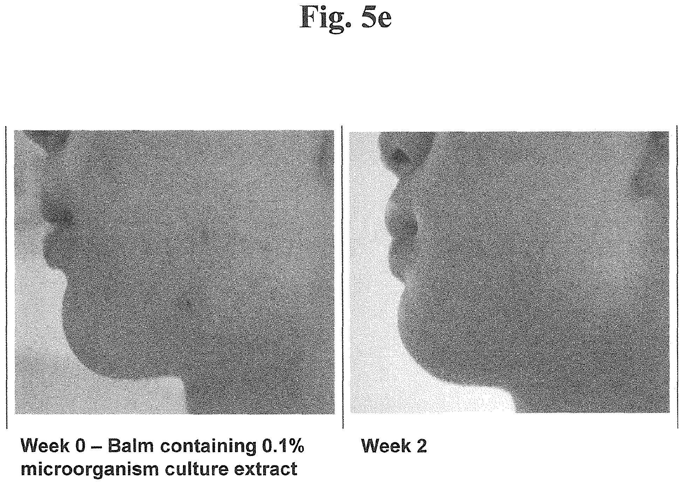

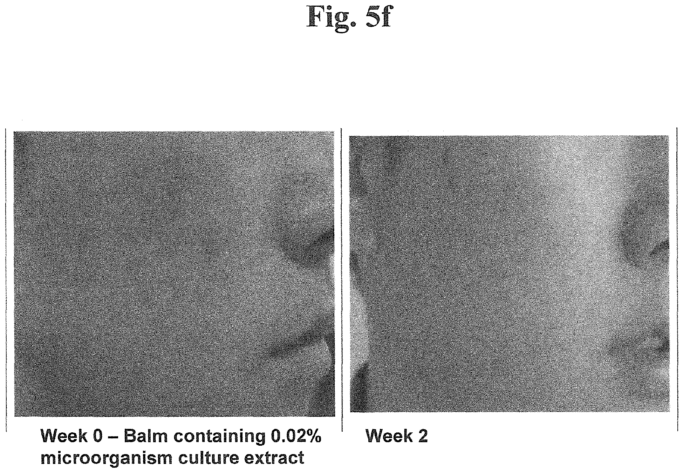

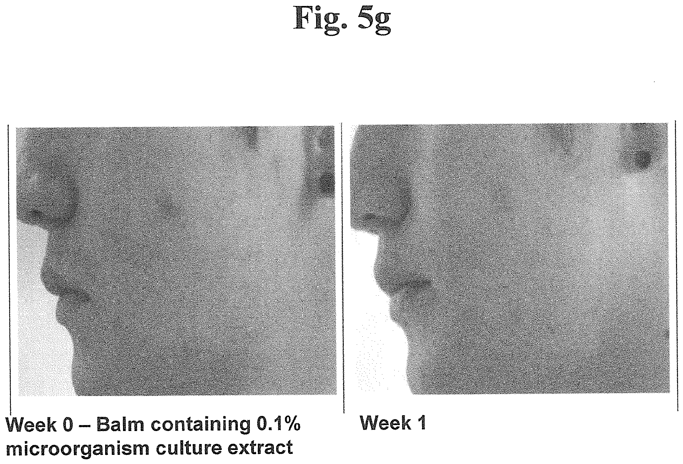

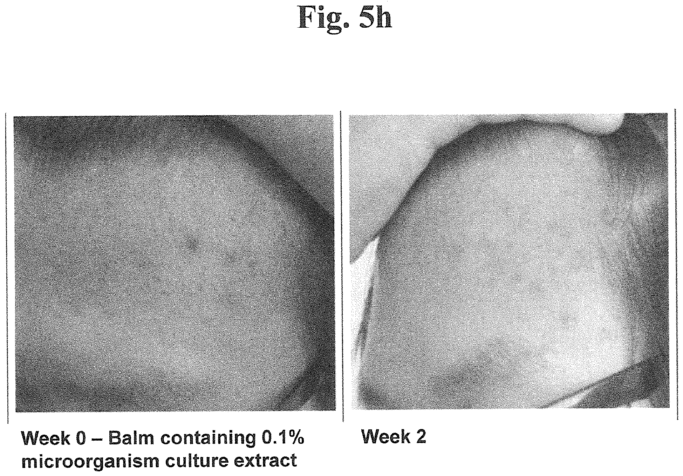

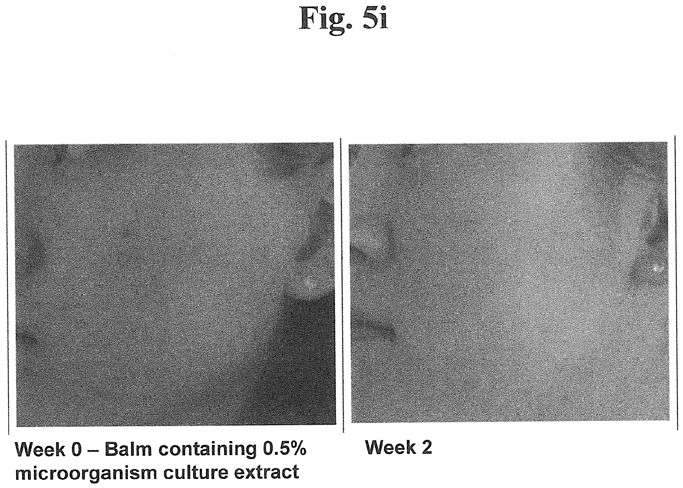

At day 0 of the clinical test, the number of ongoing acnes on the lesion site, which was designated by the tester before the product was applied, was set as an initial point, and the reduction degree by naked eyes at such a lesion site two weeks after testing was digitalized. The results verified that the subjects using the products containing the microorganism culture extract showed higher reduction rates when compared with the products not containing the microorganism culture extract. It was verified that the highest reduction rate was showed in the subjects using, especially, a cream type product containing a 0.02% microorganism culture extract, and next, a high reduction rate was showed in the subjects using a cream type product containing a 0.1% microorganism culture extract (Fig. 4). Based on these results, it can be considered that the natural cosmetic products containing the microorganism culture extract prepared by the present inventors are helpful in the reduction of acne. The image photographing results of the subjects are shown in Figs. 5a to 5i.

**[Table 14]**

| Overall effectiveness evaluation | | | |
|---|---|---|---|
| Subject No. | Evaluatioin | Subject No. | Evaluation |
| 1 | 4 | 15 | 4 |
| 2 | 3 | 16 | 4 |
| 3 | 4 | 17 | 3 |
| 4 | 5 | 18 | 4 |
| 5 | 4 | 19 | 4 |
| 6 | 3 | 20 | 4 |
| 7 | 3 | 21 | 3 |
| 8 | 3 | 22 | 3 |
| 9 | 3 | 23 | 4 |
| 10 | 5 | 24 | 4 |
| 11 | 3 | 25 | 4 |
| 12 | 3 | 26 | 5 |
| 13 | 4 | 27 | 4 |
| 14 | 4 | Average | 3.7 |

| | | | |
|---|---|---|---|
| 5: Symptoms appeared, 4: Symptoms generally distinctively improved, 3: Symptoms generally slightly improved, 2: Symptoms not changed compared with before test, 1: Symptoms worse than before test | | | |

**[Table 15]**

| Product satisfaction evaluation results after use | | | |
|---|---|---|---|
| Subject No. | Evaluatioin | Subject No. | Evaluation |
| 1 | 3 | 15 | 5 |
| 2 | 4 | 16 | 3 |
| 3 | 4 | 17 | 4 |
| 4 | 5 | 18 | 5 |
| 5 | 4 | 19 | 4 |
| 6 | 4 | 20 | 4 |
| 7 | 4 | 21 | 4 |
| 8 | 3 | 22 | 4 |
| 9 | 3 | 23 | 4 |
| 10 | 5 | 24 | 4 |
| 11 | 2 | 25 | 3 |
| 12 | 4.5 | 26 | 5 |
| 13 | 4 | 27 | 4 |
| 14 | 4 | Average | 3.9 |

| | | | |
|---|---|---|---|
| 1: Uncertain, 2: Unsatisfactory, 3: Mediocre, 4: Satisfactory, 5: Very satisfactory | | | |

Two weeks after the clinical test, the general satisfaction of the subjects in the acne-related trouble reduction effect was evaluated as being high. The most striking changes shown in the survey evaluation and visual evaluation were skin troubles, such as pustule acne and temporary pimples. On the other hand, acne miliaris and acne scars were relatively less changed. In addition, it was verified that the reduction effect and the satisfaction were higher in the subject using cream dosage form of products rather than balm dosage form of products.

It could be seen through clinical test results, visual evaluation, survey evaluation, and image photographing for two weeks, that the natural cosmetic products containing the microorganism culture extract prepared by the present inventors are helpful in the reduction of the acne-related troubles.

### 4. NMR analysis

### General observation

All the samples contained a rhamnolipid set composed of four main ingredients (two types of di-rhamnolipids and two types of mono-rhamnolipids). The content percents of di-rhamnolipids of fraction A were different in all samples (up to 30% in sample 90, and 65% or more in samples 2 and 4) (table 17). In addition, samples 0, 1, 2, and 3 contained aromatic ingredients that could be anticipated to have an effect on antibacterial activity.

### 1D-NMR analysis

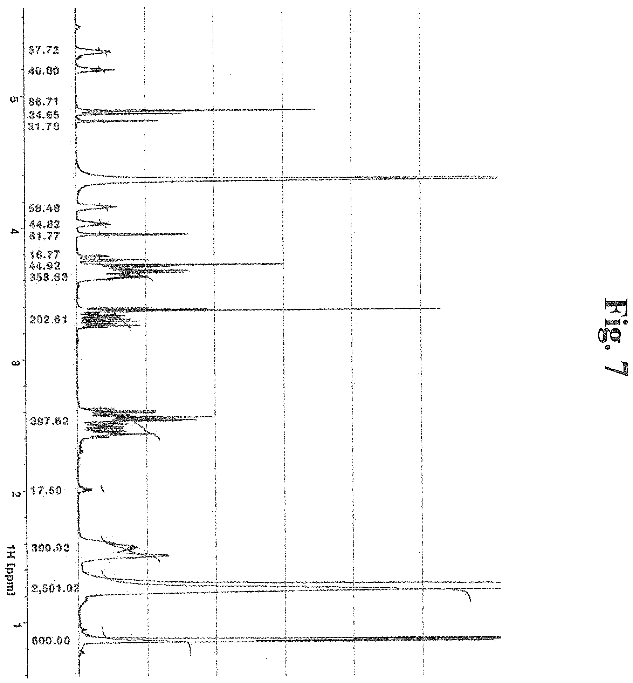

In the 1D 1H-NMR spectrum of sample 1, moieties corresponding to rhamnolipids are shown in Fig. 7. Signal intensities (integrals) are shown below the spectrum. All signal intensities were standardized according to the metal peak at 0.82 ppm, and the peaks correspond to six protons (2 methyl groups). Therefore, a single proton peak seems to have a signal intensity of approximately 100 units. For example, two groups of peaks shown at 1.4-1.5 ppm and 2.4-2.6 ppm exhibit signal intensities of -400, which are consistent with four proton signals shown at C₂H₂ / C₅H₂ and C₇H₂ / C₁₀H₂ groups, respectively. The mass ratio of mono- and di-rhamnolipids may be determined by comparing the intensities of signals obtained from lipid and rhamnose groups.

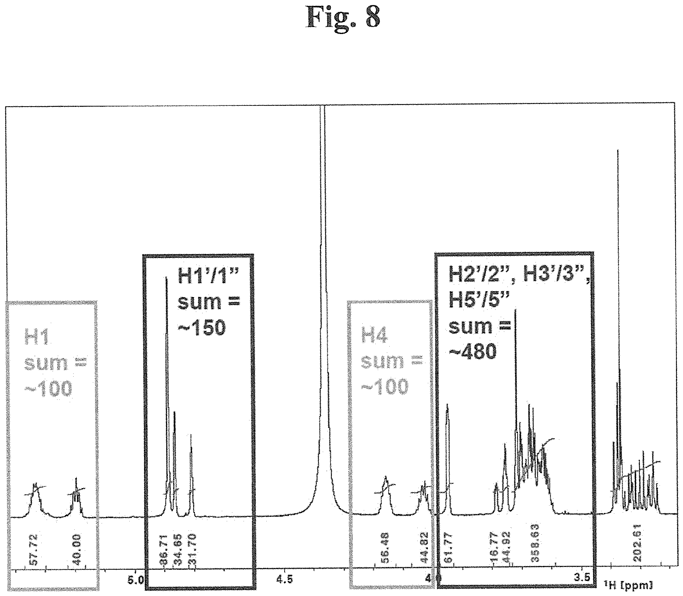

If a sample contains only mono-rhamnolipid, only a single proton from the rhamnose is consistent with one proton present in the lipid moiety. Alternatively, if a sample contains only di-rhamnolipid, the signals may be consistent with "2 -to -1". As validated in Fig. 8, H1 and H4 lipid protons (dark orange-color boxes) show the same type of signals, and the sum of the intensities thereof is approximately 100, which means that the number of this type of proton in each rhamnolipid molecule in the mixture is exactly one.

Resultantly, the sum of signal intensities obtained the H1'/1", H2'/2", H3'/3", H5'/5" rhamnose protons (blue boxes in Fig. 8) was higher than the value expected in the mono-rhamnolipid mixture. A single H1' proton per molecule in the mono-rhamnolipid mixture must show a signal having a value of 100. Three, H2' H3', and H5' protons must show signals having a value of 300. However, these signal intensities in sample 1 were measured to be approximately 150-480. It may be estimated from these signal intensity values that approximately 60% of the di-rhamnolipid was present in sample 1. By similar analysis, 1D 1H-NMAR spectra were performed on samples 0, 2, 3, and 90.

### 2D-NMR analysis

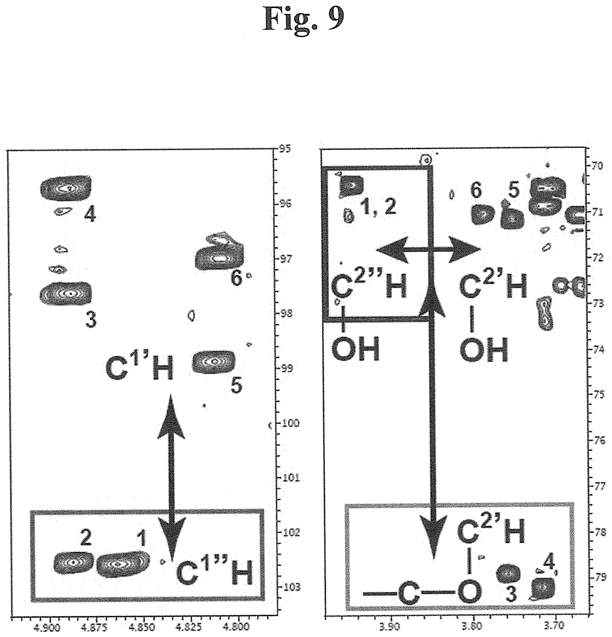

The presence of di-rhamnolipid in the samples was confirmed by 2D 1H-13C-HSQC test. The formation of β-L-Rhap (1"→2')-β-L-Rha*p* bond caused a strong NMR signal shift of C2' and C1' atoms of di-rhamnose connected via oxygen atom (structure in Fig. 6). C^{1'/1"}H and C^{2'/2"}H sections in HSQC spectrum are shown in Fig. 9. The cross-peaks shown at 102 ppm (¹³C scale, red box) are consistent with the correlation coefficient of H1"/ C1" in the di-rhamnolipid. On the other hand, the cross-peaks shown at 95-95 ppm are consistent with the correlation coefficient of H1'/ C1' in the mono-rhamnolipid (structure in Fig. 6). Two cross-peaks shown at ~79 ppm (blue boxes in Fig. 9) are consistent with two C2' atoms of the di-rhamnolipid.

### Spin system allocation

In order to measure relative densities and numbers of different rhamnolipids in the samples, the spin systems were separated using 2D NMR spectra (HSQC, TOCSY, COSY-DQF), and these were consistent with rhamnose ring structure and fatty acid tail structure of the rhamnolipid molecule. In addition, six unknown systems (systems 1-6) of the rhamnose moiety and eight systems (systems 7-14) of the fatty acid tail could be proved. The systems were previously allotted to the rhamnose moiety (first (') or second (") single ring structure in di-rhamnose), and the location of the hydroxyl fatty acid (directly or indirectly connected to rhamnose, Fig. 6) is based on the known chemical shift (part 3, 2D-NMR analysis, shown in Fig. 9). Systems 1 and 2 were allotted to the second rhamnose ring structure of the di-rhamnose, and systems 3 and 4 were allotted to the first rhamnose ring structure of the di-rhamnose. Systems 5 and 6 are consistent with the rhamnose ring structure of the mono-rhamnolipid. Systems 7 and 8 as well as minimum ingredients 9 and 10 thereof were allotted to the second fatty acid of the lipid (not directly connected to the rhamnose moiety). Resultantly, systems 11-14 were allotted to the first fatty acid chain, and directly connected to the rhamnose moiety.

### Spin system connection

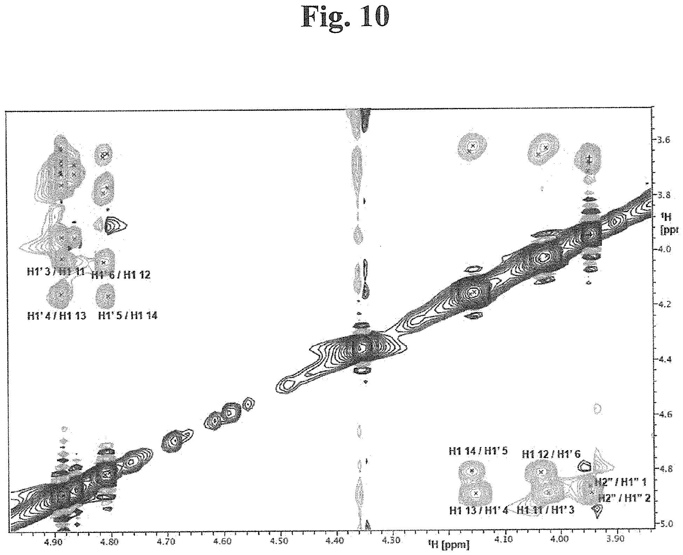

After the allotment of the spin systems for structural moieties of the rhamnolipid molecule, several possible methods for connecting the spin systems into the rhamnolipid were conducted. In order to sense the dipole-dipole interaction through the surfaces of the protons present on the rhamnose ring structure and the protons of the fatty acid, 2D NMR ROESY test was conducted. As anticipated, the cross-peaks of H1 proton of rhamnose systems 3-6 and H1 protons of fatty acid systems 11-14 were sensed (Fig. 10). This test allows the connection between the spin system allotted to the first ring structure or a single structure of rhamnose and the spin system allotted to the fatty acid.

No cross-peaks were observed between H1 protons of systems 1 and 2 allotted to the protons of the second ring structure in the di-rhamnose and the fatty acid. Ultimately, the spin systems were summarized as four rhamnolipid molecules I-IV (table 16).

**[Table 16]**

| Molecule | Rhamnose moieties / spin systems | | | | Lipid moieties / spin systems | | |
|---|---|---|---|---|---|---|---|
| | β-L-Rha*p*-1" | Linker | β-L-Rha*p*-1' | Linker | Fatty acid 1 | Liner | Fatty acid 2 |
| I | 1 (2) | 1"-O-2' | 3 | 1-O-1 | 11 | 2-C^{O}- -O-4 | 8 |
| II | 1 (2) | | 4 | | 13 | | 7 |
| III | | | 5 | | 14 | | 7 (8) |
| IV | | | 6 | | 12 | | 7 (8) |

### Relative densities of molecules I-IV in NMR sample

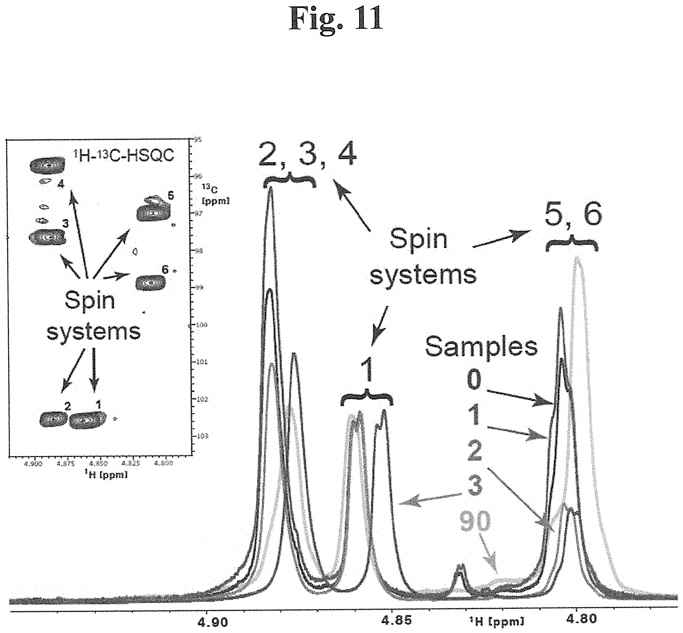

Preliminary analysis of 1D 1H-NMR spectra clearly shows relative densities of rhamnose in the samples (Fig. 11). The same results were observed in the fatty acid systems. The relative densities of molecules I-IV were estimated using signal intensities, and the signal intensities were measured by 1D 1H NMR spectra in order to obtain well separated signals allotted to the H1/H1" and H2/H2" rhamnose protons and H1 and H4 fatty acid protons. The analysis was summarized in table 16.

As a result of NMR analysis of biosurfactant R1 extracted from the microorganism (P. aeruginosa) culture, biosurfactants R2 and R3 obtained by changing culturing and extraction manners with respect to the same P. *aeruginosa,* and biosurfactant R90 containing 90% of rhamnolipids, purchased from SIGMA-ALDRICH, it was found that all the respective extracts and purchased R90 contained rhamnolipids. These results validated that R1 extracted from the microorganism (*P. aeruginosa*) culture contains rhamnolipid-based biosurfactant (Fig. 11 and table 16). In addition, it could be seen through NMR analysis that five samples contained rhamnolipids and the content percentages thereof were different. In addition, the rhamnolipids may probably be associated with other biological studies.

**[Table 17]**

| Molecule | di-Rhamnolipid | mono-Rhamnolipid | Population in sample | | | | |
|---|---|---|---|---|---|---|---|
| | | | 0 | 1 | 2 | 3 | 90 |
| I | {1/2}-3-O-11-8 | | 24 | 33 | 15 | 19 | 35 |
| II | {1/2}-4-O-13-7 | | 25 | 33 | 52 | 50 | |
| | | | | | | | |
| III | | 5-O-14-{7/8} | 31 | 16 | 25 | 22 | 47 |
| IV | | 6-O-12-{7/8} | 20 | 18 | 8 | 9 | 18 |

### 5. Antioxidative effect of Pseudomonas aeruginosa culture extract

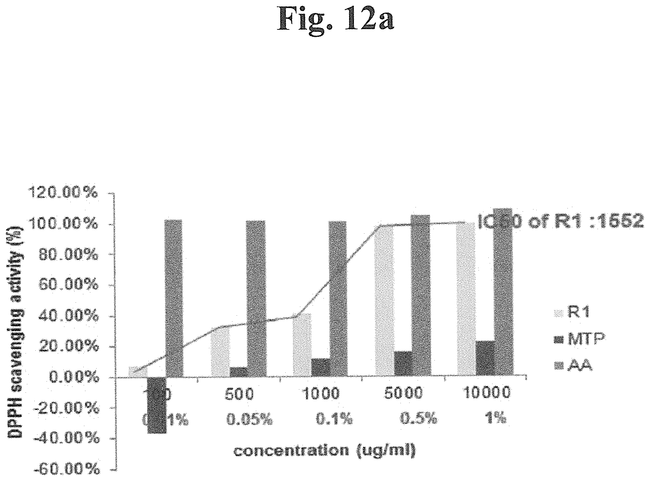

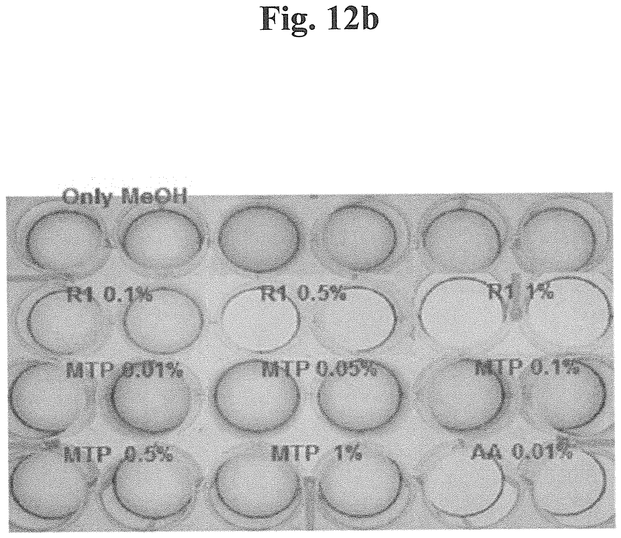

In order to investigate the antioxidative effect of *Pseudomonas aeruginosa* culture extract R1, DPPH scavenging activity test was conducted. As a result, R1 showed the same level of antioxidative effect as ascorbic acid (AA), which is a representative ingredient that has an antioxidative effect at a concentration of 0.5% or more. On the other hand, the methyl paraben (MTP) exhibited a significantly low antioxidative effect at the same concentration when compared with R1. It is considered on the basis of these results that *Pseudomonas aeruginosa* culture extract R1 having an antioxidative effect can be applied as a preservative (Fig. 12).

### 6. Search of active ingredients causing antibiotic effect of Pseudomonas aeruginosa culture extract

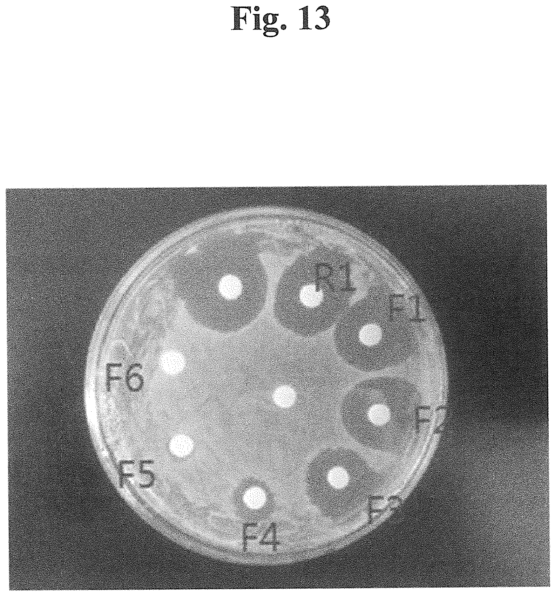

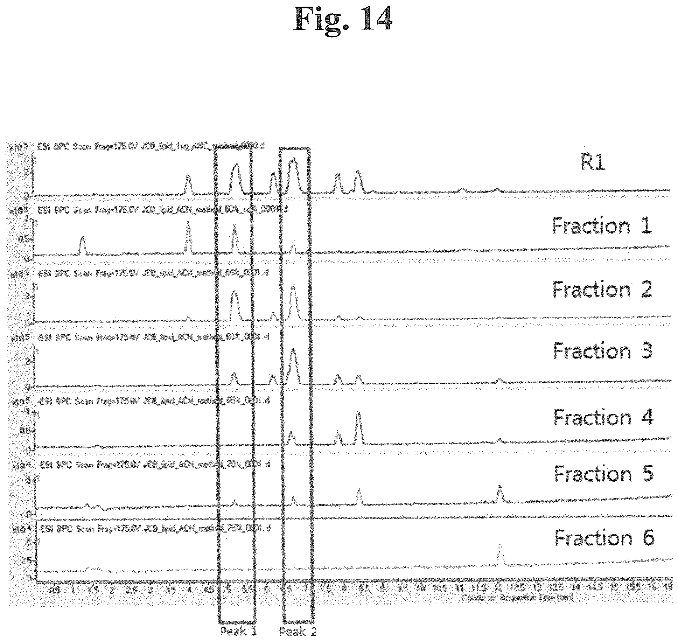

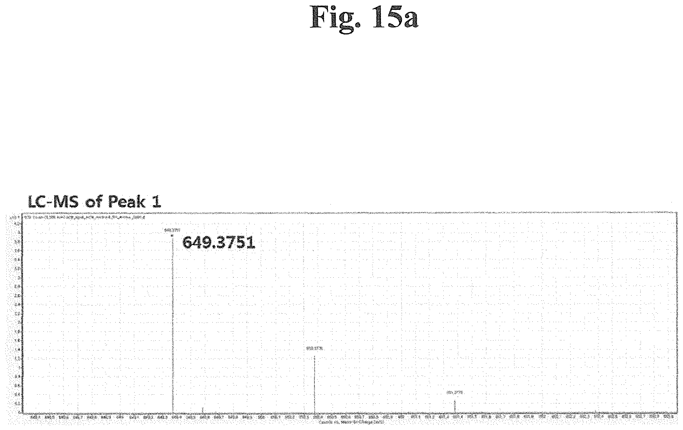

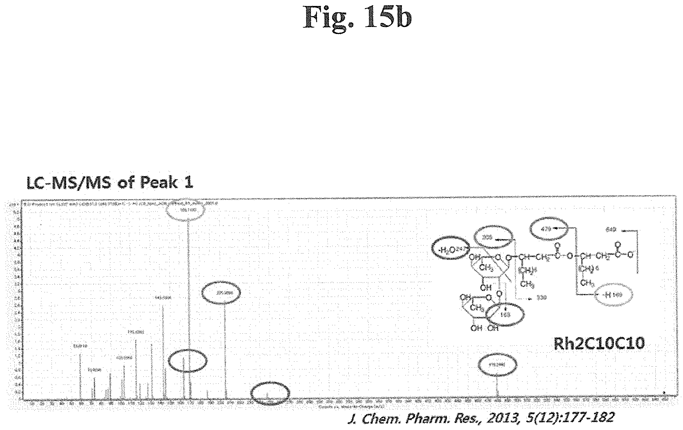

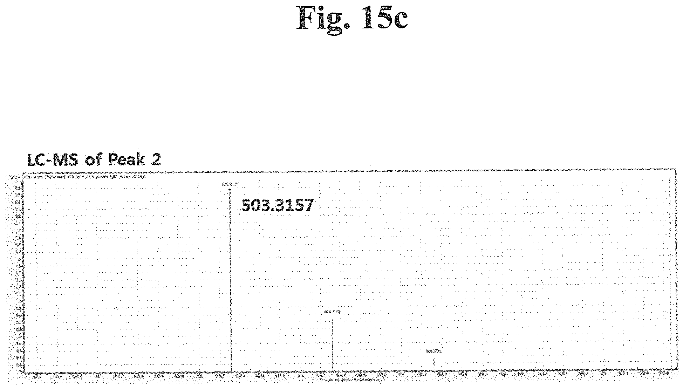

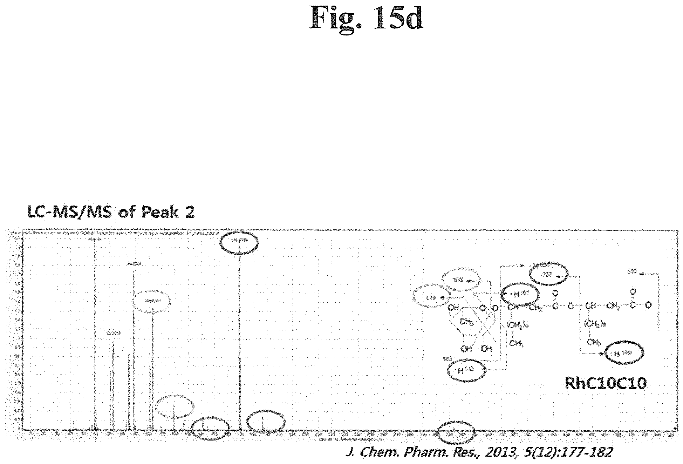

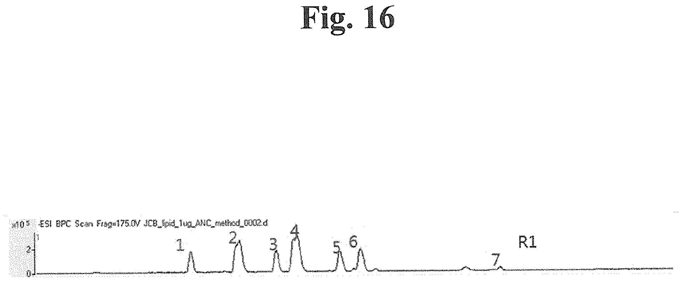

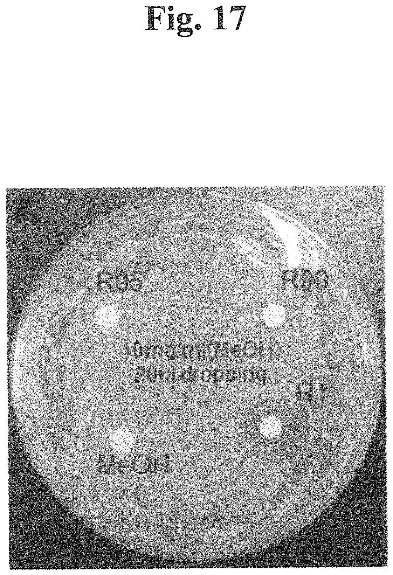

A mixture state of R1 extracted from the microorganism (P. *aeruginosa)* culture was separated to obtain fraction 1, fraction 2, fraction 3, fraction 4, fraction 5, and fraction 6. As a result of testing antibacterial activity of each of the obtained samples against S. *aureus,* fraction 1, fraction 2, and fraction 3 exhibited antibacterial activity corresponding to R1; fraction 4 and fraction 5 exhibited a relatively weaker antibacterial activity than R1; and fraction 6 did not exhibit antibacterial activity (Fig. 13). All the samples were subjected to LC analysis, and as a result, two common peaks, which are high in fraction 1, fraction 2, and fraction 3 and are gradually reduced in fraction 4 and fraction 5, were found, and the two peaks were named peak 1 and peak 2 (Fig. 14). In connection with the antibiotic activity test, the presence or absence of peak 1 and peak 2 were considered to determine antibacterial activity. As a result of MS and MS/MS tests of peak 1 and peak 2, peak 1 was analyzed as a di-rhamnolipid having a molecular weight of 650 and a structure of Rha-Rha-C10-C10, and peak 2 was analyzed as a mono-rhamnolipid having a molecular weight of 504 and a structure of Rha-C10-C10 (Figs. 15a-15d). It was verified that R1 has various structures of rhamnolipids (Fig. 16 and table 18), and of them, the rhamnolipids having structures of Rha-Rha-C10-C10 and Rha-C10-C10 are active ingredients having antibacterial activity. In addition, the antibacterial activities of rhamnolipids (R90, R95) extracted from the culture of *P. aeruginosa* purchased from SIGMA-ALDRICH KOREA against S. *aureus* were significantly low compared with R1 (Fig. 17).

**[Table 18]**

| MS of main peaks (R1) | | | | | | | |
|---|---|---|---|---|---|---|---|
| Number | Name | Symbol | Formuₗa (DB) | Avg Mass | Base Peak | RT | Vol % |
| 1 | Di-rhamnolipid | Rha-Rha-C10-C8 | C30H54O13 | 622.715 | 621.3529 | 4.059 | 5.45 |
| 2 | Di-rhamnolipid | Rha-Rha-C10-C10 | C32H58O13 | 650.7773 | 649.3865 | 5.289 | 14.22 |
| 3 | Di-rhamnolipid | Rha-Rha-C10-C12:1 | C34H60O13 | 676.7992 | 675.4063 | 6.266 | 6.33 |
| 4 | Mono-rhamnolipid | Rha-C10-C10 | C26H48O9 | 504.6391 | 503.3306 | 6.772 | 16.66 |
| 5 | Mono-rhamnolipid | Rha-C10-C12:1 | C28H59O9 | 530.6705 | 529.342 | 7.916 | 6.29 |
| 6 | Mono-rhamnolipid | Rha-C10-C12 | C28H52O9 | 532.6893 | 531.3579 | 8.451 | 7.74 |
| 7 | 2-Nor-1,3-seco-1α,25-dihydroxyvitamin D3 | | C26 H44 O3 | 404.5802 | 403.3085 | 12.059 | 1.36 |

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A preservative composition containing a *Pseudomonas aeruginosa* culture extract as an active ingredient.

2. An antibiotic composition containing a *Pseudomonas aeruginosa* culture extract as an active ingredient.

3. A composition for preventing or treating acne, containing a *Pseudomonas aeruginosa* culture extract as an active ingredient.

4. An antioxidative composition containing a *Pseudomonas aeruginosa* culture extract as an active ingredient.

5. The composition of any one of claims 1 to 4, wherein the *Pseudomonas aeruginosa* culture extract is obtained by adding a hydrochloric acid solution to a *Pseudomonas aeruginosa* culture to form a precipitate through precipitation and then adding ethyl ether to the precipitate.

6. The composition of any one of claims 1 to 4, wherein the *Pseudomonas aeruginosa* culture extract contains rhamnolipid.

7. The composition of claim 6, wherein the rhamnolipid is di-rhamnolipid represented by chemical formula 1 or mono-rhamnolipid represented by chemical formula 2.

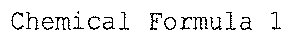

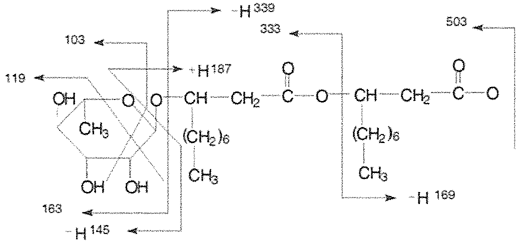

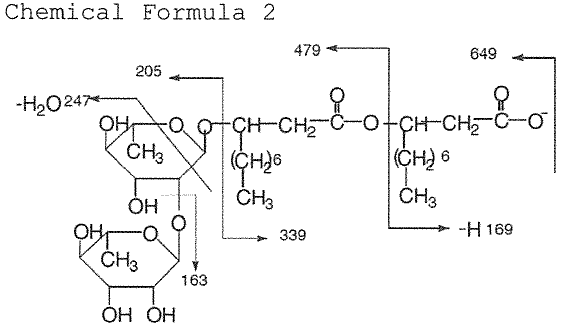

8. The composition of any one of claims 1 to 4, wherein the composition contains 0.005-1.0 parts by weight of the *Pseudomonas aeruginosa* culture extract on the basis of 100 parts by weight of the entire composition.

9. The composition of any one of claims 1 to 4, wherein the composition exhibits an antibacterial activity against at least one microorganism selected from the group consisting of *Staphylococcus spp., Bacillus spp., Pseudomonas spp., Candida spp., Propionibacterium spp., Streptococcus spp., Proteus spp., Corynebacterium spp., Enterococcus spp., Klebsiella spp.,* and *Escherichia coli.*

10. The composition of claim 1, wherein the composition is a cosmetic composition.

11. The composition of claim 2, wherein the composition is a food composition.

12. The composition of claim 3, wherein the composition is a composition for external application to skin.

13. The composition of claim 12, wherein the composition for external application to skin is formulated in one dosage form selected from solution, suspension, emulsion, paste, gel, cream, lotion, powder, soap, surfactant-containing cleansing, oil, powder foundation, emulsion foundation, wax foundation, and spray.

14. A preservation method comprising administering a *Pseudomonas aeruginosa* culture extract to a subject.

15. An antibiotic method comprising administering a *Pseudomonas aeruginosa* culture extract to a subject.

16. A method for preventing or treating acne, the method comprising administering a *Pseudomonas aeruginosa* culture extract to a subject.

17. A method for antioxidation, the method comprising administering a *Pseudomonas aeruginosa* culture extract to a subject.
